# EUROPÄISCHE PATENTSCHRIFT

(11) **EP 1 212 439 B1**
(45) Veröffentlichungstag und Bekanntmachung des Hinweises auf die Patenterteilung: **29.04.2009**
(21) Anmeldenummer: 00954593.0
(22) Anmeldetag: 05.08.2000
(51) Int. Cl.: C12N 15/82, C12N 15/29, C07K 14/415, A01H 5/00

(54) **PFLANZEN MIT VERÄNDERTEM AMINOSÄUREGEHALT UND VERFAHREN ZU DEREN HERSTELLUNG**
PLANTS HAVING ALTERED AMINO ACID CONTENTS AND METHOD FOR THE PRODUCTION THEREOF
PLANTES A TENEUR MODIFIEE EN ACIDES AMINES ET LEUR PROCEDE DE PRODUCTION

(30) Priorität: 15.09.1999 DE 19944212
(43) Veröffentlichungstag der Anmeldung: 12.06.2002
(73) Patentinhaber: BASF Plant Science GmbH, 67056 Ludwigshafen (DE)
(72) Erfinder: REINDL, Andreas, D-68199 Mannheim (DE); GEIGENBERGER, Peter, Ludwig, D-69221 Dossenheim (DE); NEUHAUS, Horst-Ekkerhard, 67659 Kaiserslautern (DE); GRAEVE-KAMPFENKEL, Karlheinz, D-55246 Mainz-Kostheim (DE); MÖHLMANN, Torsten, D-67657 Kaiserslautern (DE); TJADEN, Joachim, D-67661 Kaiserslautern (DE)
(74) Vertreter: Fitzner, Uwe
(86) Internationale Anmeldenummer: PCT/EP2000/007625
(87) Internationale Veröffentlichungsnummer: WO 2001/020009

(56) Entgegenhaltungen:
- WO-A-94/10320
- WO-A-99/58654
- TJADEN JOACHIM ET AL: "Altered plastidic ATP/ADP-transporter activity influences potato (Solanum tuberosum L.) tuber morphology, yield and composition of tuber starch." PLANT JOURNAL, Bd. 16, Nr. 5, Dezember 1998 (1998-12), Seiten 531-540, XP000960526 ISSN: 0960-7412
- KAMPFENKEL KARLHEINZ ET AL: "Molecular characterization of an Arabidopsis thaliana cDNA encoding a novel putative adenylate translocator of higher plants." FEBS LETTERS, Bd. 374, Nr. 3, 1995, Seiten 351-355, XP000960469 ISSN: 0014-5793 & DATABASE EMBL [Online] Accession No. Z49227, 3. November 1995 (1995-11-03) KAMPFENKEL, K.K.: "A. thaliana mRNA for adenine nucleotide translocase"

## Beschreibung

Die vorliegende Erfindung beschreibt transformierte Pflanzen und deren Nachkommen, die in den regulativen Sequenzen und/oder der Genkopienzahl des ATP/ADP-Translokator-Gens derart verändert sind, daß sie gegenüber einer nicht transformierten Pflanze eine bis mehrere Aminosäuren gleichzeitig in veränderten Mengen aufweisen. Ferner betrifft die vorliegende Erfindung ein Verfahren zur Herstellung dieser Pflanzen sowie deren Verwendung als Nutzpflanze oder in Bereichen der Futtermittelindustrie.

Menschen und Tiere können lediglich 11 der 20 Aminosäuren synthetisieren und sind deshalb auf eine Aufnahme der 9 sogenannten essentiellen Aminosäuren durch die Nahrung angewiesen. Die Nahrungsgrundlage von Menschen und Vieh basiert zum größen Teil auf pflanzlichen Komponenten. Zu den essentiellen Aminosäuren gehören Lysin, Tryptophan, Valin, Leucin, Isoleucin, Methionin, Threonin, Phenylalanin und Histidin.

Ein Problem entsteht durch die oftmals nur sehr geringen Konzentrationen dieser Aminosäuren in Nahrungspflanzen. Aus diesem Grund werden häufig Körnermischungen und Gemüse-basierten Nahrungsmitteln mit synthetisch hergestellten Aminosäuren supplementiert, um den Nährwert dieses Futters zu erhöhen.

In der Vergangenheit wurden zahlreiche Wege beschritten, um die Mengen an freien, d.h. nicht in Proteinen gebundenen Aminosäuren zu erhöhen. Diese Versuche konzentrierten sich jedoch vor allem auf die klassische Züchtung sowie auf die Selektion von Mutanten.

In der jüngeren Vergangenheit wurde zunehmend versucht, die Mengen an essentiellen Aminosäuren durch die Anwendung molekulargenetischer Techniken zu erhöhen. WO 97 28247, WO 98 13506 und WO 9735023 beschreiben erste Versuche, die sich auf die heterologe Expression eines samenspezifischen Speicherproteins erstrecken, welches reich an Lysin oder Methionin ist. Nachteilig ist hierbei die Speicherung der Aminosäuren in Proteinen, d.h. es handelt sich auch hier um eine Erhöhung an gebundenen Aminosäuren.

Ferner sind zahlreiche Versuche zur direkten Beeinflussung der Aminosäure-Biosynthese bekannt. Hier wurden einzelne Gene kodierend für bestimmte Aminosäure-Biosyntheseenzyme in Pflanzen überexpremiert, resultierend in einer Erhöhung des jeweiligen Biosyntheseendprodukts.

Alternativ dazu wurde außerdem versucht, Enzyme in ihren Reaktionskinetiken zu beeinflussen. Hierbei stellt insbesondere die sogenannte Produkthemmung von Enzymen ein Problem dar. Beispielsweise beschreiben Shaul und Galili (1993); Plant Mol Biol 23: 759-768) bzw. Falco et al (1995; Bio/Technology 13: 577-582) Pflanzen, die freies Lysin überproduzieren, einhergehend mit einer Abnahme von freiem Threonin. Verantwortlich hierfür ist die Aspartatkinase, dem ersten Enzym der Biosynthese der von Aspartat abstammenden Aminosäure, welche durch Lysin allosterisch gehemmt wird. Zur Umgehung dieser Feedback-Hemmung wurden gentechnisch modifizierte Gene, der Aspartatkinase in Pflanzen überexpremiert (WO 94 25605). Diese veränderte Aspartatkinase verfügt über eine stark verringerte Feedback-Inhibition durch Lysin und Threonin, was zu einer Zunahme an Lysin führt. Diese für die Feedback-Hemmung durch Lysin insensitive Aspartatkinase wurden außerdem zusammen mit anderen Biosyntheseenzymen überexpremiert. Ensprechende Versuche wurden in Corynebakterien durchgeführt (1991, Applied an Environmental Microbiology 57: 1746-1752). In diesen Bakterien kommt es jedoch neben einer Zunahme von Lysin auch zu einer starken Abnahme in der Wachstumsgeschwindigkeit, was sich wiederum negativ auf die Lysinbilanz auswirkt.

Versuche mit Pflanzen, die sowohl eine Feedback-insensitive Aspartatkinase als auch eine Feedback-insensitive Dihydropicolinat-Synthase besitzen, sind bei Shaul und Galili (1993; Plant Mol Biol 23: 759-768) beschrieben. Beide Enzyme nehmen eine Schlüsselposition in der Aminosäure-Biosynthese ein. Die Überexpression dieser "Flaschenhals"-Enzyme führte jedoch nicht zu der erhofften Erhöhung beider Aminosäuren, Lysin und Threonin. Vielmehr konnte nur der Gehalt an freiem Lysin erhöht werden, wobei gleichzeitig der Gehalt an freiem Threonin drastisch sank.

Über die Überexpression von einem oder zwei Aminosäure-Biosynthesegenen hinaus beschreiben WO 98 56935, EP 0 854 189 und EP 0 485 970 Mulit-Gen-Ansätze mit dem Ziel, die Mengen einer oder mehrerer Aminosäuren gleichzeitig in einer Pflanze zu beeinflussen. Dies setzt die genetische Modifikation einer Pflanze hinsichtlich mehrerer Gene voraus. D.h. es wäre notwendig, eine mehrfach transgene Pflanze herzustellen. Diese Verfahren sind jedoch sehr aufwendig. Außerdem bergen solche massiven Eingriffe in das pflanzliche Erbmaterial zunehmend Risiken unvorhersehbarer Nebenreaktionen in sich.

Aufgabe der vorliegenden Erfindung ist es daher, ein Verfahren zur Herstellung von transgenen pflanzen zur Verfügung zu stellen, die die zuvor genannten Nachteile nicht aufweisen.

Überraschenderweise wird dies erfindungsgemäß durch die Bereitstellung eines Verfahrens zur Herstellung einer transformierten Pflanze erreicht, die in den regulativen Sequenzen und/oder der Genkopienzahl eines ATP/ADP-Translokator-Gens derart verändert ist, daß sie gegenüber einer entsprechenden nicht transformierten Pflanze eine bis mehrere Aminosäuren in veränderten Mengen aufweist.

Die transformierten Pflanzen zeichnen sich dadurch aus, daß sie überwiegend eine oder mehrere essentielle Aminosäure(n) in veränderten Mengen aufweisen.

Insbesondere weisen die Pflanzen eine oder mehrere essentielle Aminosäure(n) auf, deren Gehalt gegenüber der nicht transformierten Pflanzen gesteigert ist.

Bei den transformierten Pflanzen handelt es sich erfindungsgemäß um Nutzpflanzen, bevorzugt wirtschaftlich relevante Pflanzen, wie beispielsweise Kartoffeln oder Mais. Die vorliegende Erfindung jedoch nicht auf diese Gattungen beschränkt.

Die vorliegende Erfindung bezieht sich dabei auf ein Verfahren zur Herstellung der zuvor genannten transformierten Pflanzen, deren Samen und Nachkommen als auch auf von diesen transformierten Pflanzen abgeleitetes Gewebe, Zellen oder fortpflanzungsfähiges Material.

In einer Ausführungsvariante der vorliegenden Erfindung, bei der erfindungsgemäß das Gen kodierend für den ATP/ADP-Translokator in Kartoffeln überexprimiert wird, wird eine Erhöhung ernährungsphysiologisch und wirtschaftlich interessanter Aminosäuren, wie Lysin, Methionin, Threonin, Valin, Tryptophan, Histidin, Isoleucin und Leucin erreicht.

In der mit Linie 98 bezeichneten transformierten Pflanze ist die Menge an freiem Lysin um 28% gesteigert, in der mit Linie 62 bezeichneten transgenen Pflanze liegt die Erhöhung der freien Lysinmenge bei 25,75%. Überraschenderweise wird bereits durch eine nur 50%ige Steigerung der ATP/ADP-Translokator-Aktivität in den Pflanzen eine mindestens 25%ige Erhöhung des Lysingehaltes erreicht. Ferner ist in der Linie 98 die Menge an Methionin um 11% erhöht. Neben erhöhten Lysin- und Methioninmengen liegen ebenfalls erhöhte Mengen der essentiellen Aminosäuren Valin (12 % in Linie 98), Tryptophan (50 % in Linie 98), Threonin (12,5 % in Linie 98), Histidin (23,5 % in Linie 98 und 20 % in Linie 62), Isoleucin (25 % in Linie 98) und Leucin (40 % in Linie 98) vor.

Eine Überexpression des ATP/ADP-Translokators in Antisense-Orientierung führt entsprechend zu einer Erniedrigung der Aminosäuremengen in den jeweiligen transformierten Pflanzen, die mit Linie 594 und 595 bezeichnet sind. Für Lysin findet man hier nur noch etwa ein Viertel der Wildtyp-Lysinmenge, bei Methionin ist es noch etwa maximal ein Achtel der Wildtyp-Methioninmenge.

Eine Übersicht über das Spektrum an Aminosäuren im Wildtyp der Kartoffel Solanum tuberosum und in transformierten Kartoffelpflanzen ist in Tabelle 1 zusammengefaßt. In dieser Ausführungsvariante der Erfindung ist die Gesamtmenge an freien Aminosäuren in den transformierten Kartoffelpflanzen gegenüber dem Wildtyp um etwa 7% erhöht.

Ein besonderer Vorteil der vorliegenden Erfindung ist es, daß durch die gesteigerte Expression eines einzigen Gens, nämlich des ATP/ADP-Translokators, eine spezifische Erhöhung mehrerer und überwiegend essentieller Aminosäuren gleichzeitig erreicht werden kann.

Die transformierte Pflanze zeichnet sich dadurch aus, daß sie eine erhöhte Transportkapazität für ATP in die Chloroplasten-Hüllmembran aufweist.

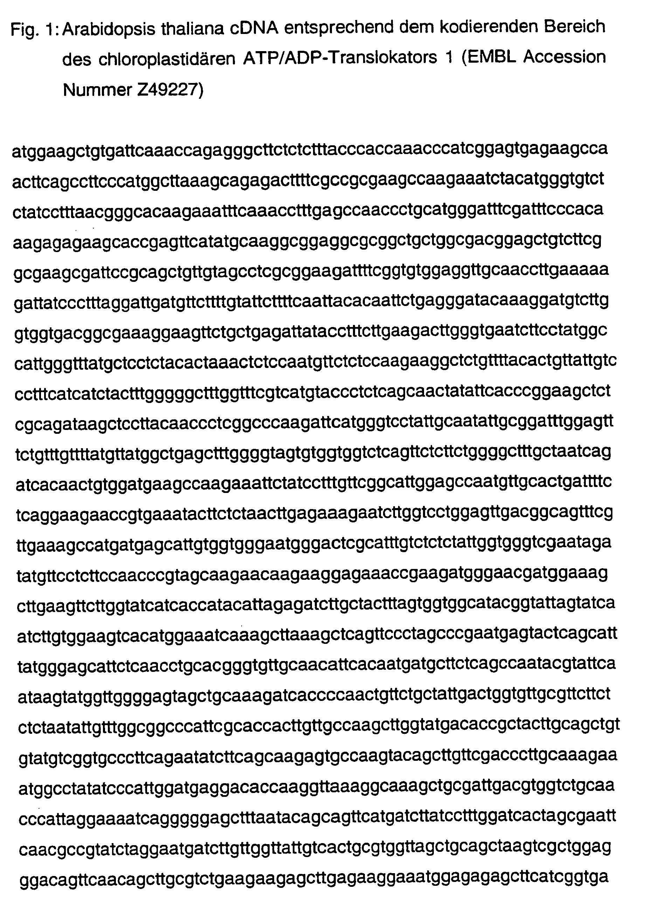

Gegenstand der Erfindung ist ferner ein ATP/ADP-Translokator-Gen zum Einsatz in eine der zuvor beschriebenen Pflanzen mit einer für die in Fig. 1 angegebenen Aminosäuresequenz kodierenden Nukleotidsequenz aus Arabidopsis thaliana (EMBL Accession Nr. Z49227).

Erfindungsgemäß ist hier der Einsatz eines jeden ATP/ADP-Translokator-Gens aus Organismen denkbar, die Chloroplasten besitzen. Bevorzugt sind Pflanzen im allgemeinen, Grünalgen oder Moose.

Das ATP/ADP-Translokator-Gen ist normalerweise in der inneren Chloroplasten-Hüllmembran lokalisiert. Dort ist es für den Antiport, also den entgegengesetzt gerichteten Transport von ATP und ADP zuständig, indem es chloroplastidäres ADP im Austausch gegen ATP ins Cytosol exportiert. Durch die erhöhte Aktivität dieses ATP/ADP-Translokators wird die Menge an ATP im Chloroplasten erhöht (Neuhaus et al., 1997, The Plant Journal 11: 73-82). Tjaden et al, 1998, Plant Journal 16: 531-540 konnte zeigen, daß die Aufnahme von ATP in Chloroplasten von Kartoffeln durch Überexpression des ATP/ADP-Translokators um durchschnittlich 50 % über der Aufnahmekapazität des Wildtyps liegt. Diese vermehrt zur Verfügung stehenden energiereichen ATP Moleküle können zur gesteigerten Biosynthese von Stärke und Fettsäuren genutzt werden, wie bei Möhlmann et al., 1994, Planta, 194: 492-497; Neuhaus et al. 1993, Plant Physiology 101: 573-578; Tjaden et al, 1998, Plant Journal 16: 531-540; WO 99/58654 beschrieben ist.

Erfindungsgemäß kann auch ein ATP/ADP-Translokator-Gen mit einer im wesentlichen gleichwirkenden natürlichen, chemisch synthetisierten, modifizierten, artifizell erzeugten Nukleotidsequenz oder mit heterologen Nukleotidsequenzen kodierend für einen ATP/ADP-Translokator oder Allelvariationen oder Isoformen davon oder mit Mischungen davon eingesetzt werden.

Gleichwirkende Sequenzen, die für ein ATP/ADP-Translokator-Gen kodieren, sind solche Sequenzen, welche trotz abweichender Nukleotidsequenz noch die gewünschten Funktionen besitzen. Gleichwirkende Äquivalente umfassen somit natürlich vorkommende Varianten der beschriebenen Sequenzen sowie künstliche, z.B. durch chemische Synthese erhaltene, an den Kodon-Gebrauch einer Pflanze angepaßte, künstliche Nukleotid-Sequenzen.

Unter einer gleichwirkenden Nukleotidsequenz versteht man insbesondere auch natürliche oder künstliche Mutationen einer ursprünglich isolierten für einen ATP/ADP-Translokator kodierenden Sequenz, welche weiterhin die gewünschte Funktion zeigt. Mutationen umfassen Substitutionen, Additionen, Deletionen, Vertauschungen oder Insertionen eines oder mehrerer Nukleotidreste. Somit werden beispielsweise auch solche Nukleotidsequenzen durch die vorliegende Erfindung mit umfaßt, welche man durch Modifikation der ATP/ADP-Translokator-Nukleotidsequenz erhält. Ziel einer solchen Modifikation kann z.B. die weitere Eingrenzung der darin enthaltenen kodierenden Sequenz oder z.B. auch die Einfügung weiterer Restriktionsenzym-Schnittstellen sein.

Gleichwirkende Nukleotidsequenzen sind auch solche Varianten, deren Funktion, verglichen mit dem Ausgangsgen bzw. Genfragment, abgeschwächt oder verstärkt ist.

Außerdem sind artifizielle DNA-Sequenzen geeignet, solange sie, wie oben beschrieben, die gewünschten Eigenschaften vermitteln. Solche artifiziellen DNA-Sequenzen können beispielsweise durch Rückübersetzung mittels Molecular Modelling konstruierter Proteine, die eine ATP/ADP-Translokator-Aktivität aufweisen oder durch in vitro-Selektion ermittelt werden. Besonders geeignet sind kodierende DNA-Sequenzen, die durch Rückübersetzung einer Polypeptidsequenz gemäß der für die Wirtspflanze spezifischen Kodon-Nutzung erhalten wurden. Die spezifische Kodon-Nutzung kann ein mit pflanzengenetischen Methoden vertrauter Fachmann durch Computerauswertungen anderer, bekannter Gene der zu transformierenden Pflanze leicht ermitteln.

Unter einer operativen Verknüpfung versteht man die sequenzielle Anordnung beispielsweise von Promotor, kodierender Sequenz, Terminator und ggf. weiterer regulativer Elemente derart, daß jedes der regulativen Elemente seine Funktion bei der Expression der kodierenden Sequenz bestimmungsgemäß erfüllen kann. Als Promotor ist grundsätzlich jeder Promotor geeignet, der die Expression von Fremdgenen in Pflanzen steuern kann. Vorzugsweise wird ein pflanzlicher Promotor oder ein Promotor, der einem Pflanzenvirus entstammt verwendet. Insbesondere bevorzugt ist der CAMV 35SPromotor aus dem Blumenkohl-Mosaik-Virus (Franck et al., Cell 21 (1980), 285-294). Dieser Promotor enthält bekanntlich unterschiedliche Erkennungssequenzen für transkriptionale Effektoren, die in ihrer Gesamtheit zu einer permanenten und konstitutiven Expression des eingeführten Gens führen (Benfey et al., EMBO J, 8 (1989),2195-2202).
Weitere zur operativen Verknüpfung bevorzugte aber nicht darauf beschränkte Sequenzen sind Transkriptionsterminatoren und Translationsverstärker, wie die 5'-Führungssequenz aus dem Tabak-Mosaik-Virus (Gallie et al., Nucl. Acids Res. 15 (1987), 8693-871 1).

Für die Verbindung der DNA-Fragmente miteinander können an die Fragmente Adaptoren oder Linker angesetzt werden. Bevorzugt können die Promotor- und die Terminator-Regionen in Transkriptionsrichtung mit einem Linker oder Polylinker, der eine oder mehrere Restriktionsstellen für die Insertion dieser Sequenz enthält, versehen werden. In der Regel hat der Linker 1 bis 10, bevorzugt 1 bis 8, besonders bevorzugt 2 bis 6 Restriktionsstellen. Im allgemeinen hat der Linker innerhalb der regulatorischen Bereiche eine Größe von weniger als 100 bp, häufig weniger als 60 bp, mindestens jedoch 5 bp. Der Promotor kann sowohl nativ bzw. homolog als auch fremdartig bzw. heterolog zur Wirtspflanze sein.

Gegenstand der Erfindung ist ferner eine Genstruktur enthaltend ein ATP/ADP-Translokator-Gen sowie mit diesem Gen operativ verknüpfte regulative Sequenzen sowie ein Vektor enthaltend ein ATP/ADP-Translokator-Gen oder eine Genstruktur wie zuvor beschrieben. Hierbei kann der Vektor zusätzliche regulative Nukleotidsequenzen, bevorzugt aus der Gruppe der Promotoren, Terminatoren oder Translationsverstärker sowie Nukleotidsequenzen für die Replikation in einer entsprechenden Wirtszelle oder zur Integration in deren Gnom enthalten.

Unter Verwendung der an sich bekannter Rekombinations- und Klonierungstechniken können die Genstrukturen in geeignete Vektoren kloniert werden, die ihre Vermehrung in Wirtszellen, wie beispielsweise Pflanzen, Pflanzengeweben oder -zellen ermöglichen. Geeignete Vektoren sind unter anderem in "Methods in Plant Molecular Biology and Biotechnology" (CRC Press), Kap. 6/7, S. 71-119 (1993) beschrieben.

Für *E. coli* als Wirtszelle sind als Klonierungsvektoren vor allem pBR332, pUC-Serien, M13mp-Serien und pACYC1 84 bevorzugt. Besonders bevorzugt sind binäre Vektoren, die sowohl in *E. coli* als auch beispielsweise in Agrobakterien replizieren können. Beispielhaft sei hierfür der pBIN19 genannt (Bevan et al., Nucl. Acids Res. 12 (1984), 871 1). Beispielhaft kann die erfindungsgemäße Genstruktur auch in den Tabak-Transformationsvektor pBIN-AR-TP eingebaut werden.

Ferner betrifft die vorliegende Erfindung ein Verfahren zur Herstellung einer wie zuvor beschriebenen transformierten Pflanze, wobei ein ATP/ADP-Translokator-Gen, eine Genstruktur oder ein Vektor der zuvor beschriebenen Art durch gentechnische Methoden in die Pflanze oder Gewebe oder Zellen davon übertragen wird. Allgemein ist dabei unter der Übertragung von DNA die Transformation von Pflanzen, -gewebe oder - zellen zu verstehen.

Geeignete Methoden zur Transformation und Regeneration von Pflanzen aus Pflanzengeweben oder Pflanzenzellen zur transienten oder stabilen Transformation sind die Protoplastentransformation durch Polyethylenglykol-induzierte DNA-Aufnahme, das biolistische Verfahren mit der Genkanone - die sogenannte particle bombardment Methode, die Elektroporation, die Inkubation trockener Embryonen in DNA-haltiger Lösung, die Mikroinjektion und der durch Agrobacterium vermittelte Gentransfer. Die genannten Verfahren sind beispielsweise in B. Jenes et al., Techniques for Gene Transfer, in: Transgenic Plants, Vol. 1, Engineering and Utilization, herausgegeben von S. D. Kung und R. Wu, Academic Press (1993), 128143 sowie in Potrykus, Annu. Rev. Plant Physiol. Plant Molec. Biol. 42 (1991), 205225) beschrieben.

Durch die vorliegende Erfindung ist es somit möglich, wirtschaftlich hochwertige Nutzpflanzen herzustellen, die sich durch einen wesentlich erhöhten Gehalt an Aminosäuren, insbesondere an essentiellen Aminosäuren, auszeichnen.

Die vorliegende Erfindung beschreibt die Verwendung der transformierten Pflanze als Nutz- oder Futterpflanze. Da in den erfindungsgemäßen Nutzpflanzen insbesondere gleichzeitig mehrere essentielle Aminosäuren in ihrem Gehalt gesteigert werden können, entfällt in vorteilhafter Weise eine kostenintensive Supplementation der Futtermittel mit Aminosäuren, die nach konventionellen Methoden bislang separat hergestellt oder gewonnen werden und dem Futter extern beigemengt werden müssen.

Ferner findet die transformierte Pflanze, deren Samen und Nachkkommen sowie Gewebe oder Zellen davon oder Extrakten daraus in Bereichen der Landwirtschaft, Futtermittelindustrie, Pharmaindustrie oder im Gesundheitswesen Anwendung.

Nachfolgend wird die vorliegende Erfindung durch Ausführungsbeispiele näher erläutert, die jedoch nicht limitierend im Sinne der Erfindung sind:

### 1. Allgemeine Klonierungsverfahren

Klonierungsverfahren wie z.B. Restriktionsspaltungen, Agarose-Gelektrophorese, Reinigung von DNA-Fragmenten, Transfer von Nukleinsäuren auf Nitrozellulose und Nylon Membranen, Verknüpfen von DNA-Fragmenten, Transformation von E.coli Zellen, Anzucht von Bakterien, Vermehrung von Phagen und Sequenzanalyse rekombinanter DNA wurden wie bei Sambrook et al (1989) (Cold Spring Harbor Laboratory Press: ISBN 0-87969-309-6) beschrieben durchgeführt.

Die verwendeten Bakterienstämme (E. coli, XL-I Blue) wurden von Stratagene (Heidelberg) oder Qiagen (Hilden) bezogen. Der zur Pflanzentransformation verwendete Agrobakterienstamm (Agrobacterium tumefaciens, C58C1 mit dem Plasmid pGV2260 oder pGV3850kan) wurde von Deblaere et al. in Nucl. Acids Res. 13 (1985), 4777 beschrieben. Alternativ können auch der Agrobakterienstamm LBA4404 (Clontech) oder andere geeignete Stämme eingesetzt werden.

Zur Klonierung können die Vektoren pUC19 (Yanish-Perron, Gene 33 (1985), 103-119) pBluescript SK-(Stratagene), pGEM-T (Promega), pZerO (Invitrogen) pBin19 (Bevan et al., Nucl. Acids Res. 12 (1984), 8711-8720) und pBinAR (Höfgen und Willmitzer, Plant Science 66 (1990), 221-230) benutzt werden.

### 2. Transformation von Agrobakterien

Die Transformation von Agrobacterium tumefaciens wurde entsprechend der Methode von Höfgen und Willmitzer (Nucl. Acid Res. (1988) 16, 9877) ausgeführt. Die Anzucht der Agrobakterien erfolgte in YEB Medium (Vervliet et al., J. Gen. Virol. (1975) 26, 33).

### 3. Sequenzanalyse rekombinanter DNA

Die Sequenzierung rekombinanter DNA-Moleküle erfolgte mit einem Laserfluoreszenz-DNA-Sequenzierer der Firma Licor (Vertrieb durch MWG Biotech., Ebersbach) nach der Methode von Sanger (Sanger et al., Proc. Natl. Acad. Sci. USA 74 (1977), 5463-5467).

### 4. Konstruktion eines Pflanzentransformationsvektor mit AATP1 in Sense-Orientierung

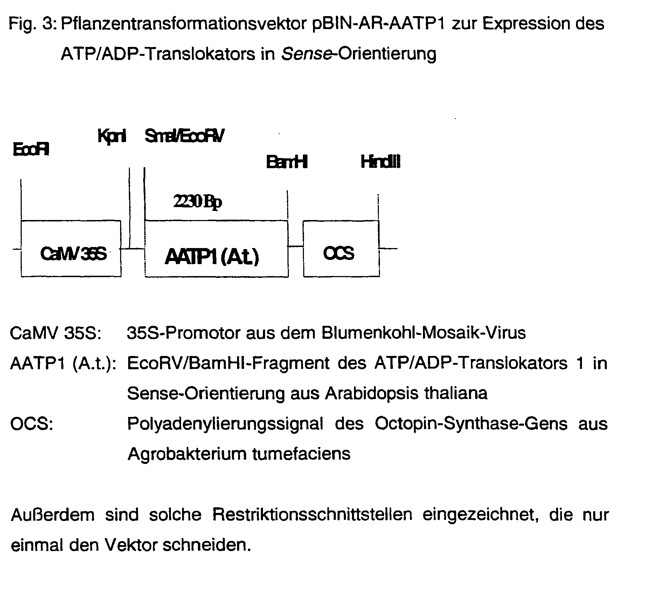

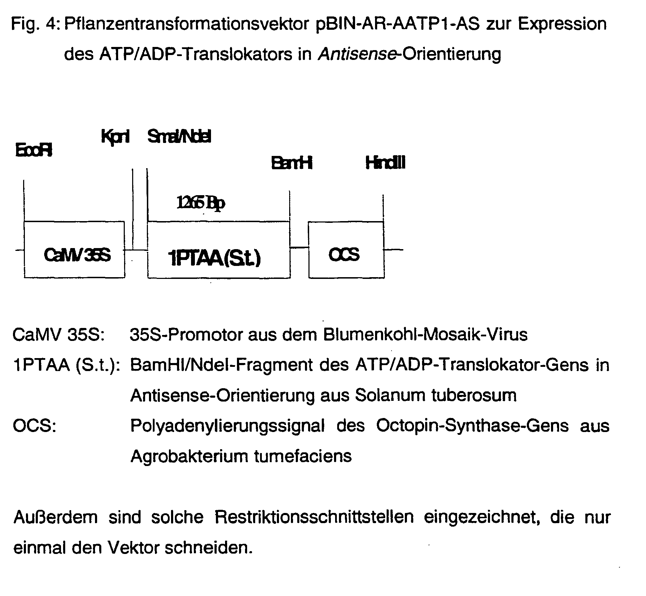

Für die Konstruktion eines Vektors zur Transformation von Pflanzen wird ein 2230 bp langes EcoRV/BamHI-Fragment der AATP1-cDNA aus Arabidopsis thaliana (Beschreibung der Klonierung von AATP1 aus Arabidopsis thaliana in Kampfenkel et al., FEBS Letters 374 (1995), 351-355 und Neuhaus et al., The Plant Journal 11: 73-82) in einen mit Smal/EcoRV und BamHI geschnittenen Vektor pBinAR (Höfgen und Willmitzer, Plant Sci. 66 (1990), 223-230) ligiert. Durch die Insertion des cDNA-Fragmentes entsteht ein Genkonstrukt, das den 35S-Promotor aus dem Blumenkohlmosaikvirus (540bp) und die proteincodierende Region des ADP/ATP-Translokators 1 aus Arabidopsis thaliana (AATP1) enthält. Das cDNA-Fragment wird in Sense-Orientierung mit dem 35S-Promotor in pBinAR fusioniert. In 3'-Richtung des inserierten AATP1-Fragments folgt das Polyadenylierungssignal des Octopin-Synthase-Gens aus Agrobakterium tumefaciens (215 bp).
Die Gesamtgröße des Plasmids pBIN AR-AATP1 (Fig. 3) beträgt ca. 14,2 kb.

### 5. Einführung des Plasmids pBINAR-ATTP1 in das Genom von Kartoffelpflanzen

Das Plasmid wird mit Hilfe von Agrobaktierium tumefaciens wie bei Rocha-Sosa et al. beschrieben (EMBO J. 8 (1989), 23-29), in Kartoffelpflanzen transferiert. Als Positivkontrolle der Transformation dienen transgene Kartoffelpflanzen, die eine Erhöhung der mRNA des plastidären ADP/ATP-Translokators 1 aufweisen. Dies wird mittels Northern Blot-Analyse nachgewiesen. Dazu wird RNA nach Standardprotokollen aus Blatt- und Knollengewebe von Kartoffelpflanzen isoliert. 50 µg RNA werden auf einem Agarosegel aufgetrennt (1,5 % Agarose, 1 x MEN-Puffer, 16,6 % Formaldehyd). Nach der Elektrophorese wird die RNA mit 20 x SSC mittels Kapillarblot auf eine Nylonmembran Hybond N (Amersham, UK) übertragen. Die RNA wird auf der Membran durch UV-Bestrahlung fixiert, die Membran in Phosphathybridisierungspuffer (Sambrook et al., a.a.O) für 2 h prähybridisiert und anschließend durch Zugabe der radioaktiv markierten Sonde für 10 h hybridisiert.

### 6. Konstruktion eines Pflanzentransformationsvektors mit AATP1 in Antisense-Orientierung

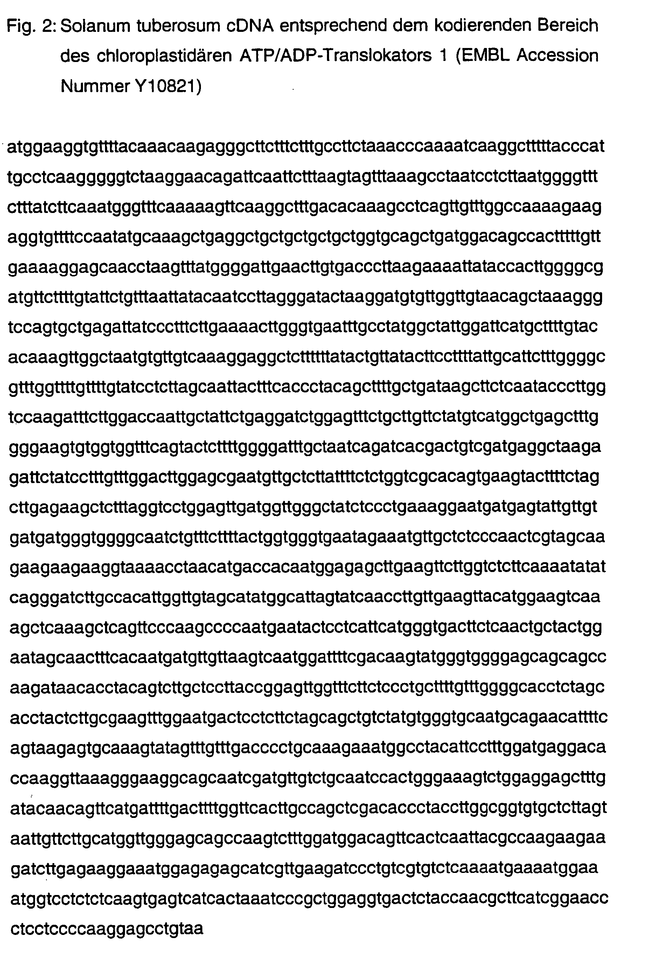

Für die Konstruktion eines Vektors zur Transformation von Pflanzen wird ein 1265 bp langes BamH/Ndel-Fragment, bei dem die Ndel-Schnittstelle mit T4-Polymerase zur blunt-end Schnittstelle aufgefüllt wird, aus der kodierenden Region der AATP1-cDNA aus S. tuberosum (Beschreibung der Klonierung von AATP1 aus Kartoffel in Tjaden et al., 1998, The Plant Journal 16: 531-540) in einen mit Smal und BamHI geschnittenen Vektor pBinAR (Höfgen und Willmitzer, Plant Sci. 66 (1990), 221-230) ligiert. Die Ndel-Schnittstelle liegt in der AATP1 cDNA, die BamHI-Schnittstelle stammt aus dem Vektor pTM1 (Tjaden et al., 1998, The Plant Journal 16: 531-540). Durch die Insertion des cDNA-Fragmentes entsteht ein Genkonstrukt, das den 35S-Promotor aus dem Blumenkohlmosaikvirus (540bp) und eine 1265 bp lange Region eines ADP/ATP-Translokators 1 aus S. tuberosum (AATP1 S.t.) in Antisense-Orientierung enthält. Das Fragment wurde mit dem 35S-Promotor in pBinAR fusioniert. In 3'-Richtung des inserierten AATP1-Fragments folgt das Polyadenylierungssignal des Octopin-Synthase-Gens aus Agrobakterium tumefaciens (215 bp).
Die Gesamtgröße des Plasmids pBIN AR-AS-AATP1 (Fig. 2) beträgt ca. 13,3 kb.

### 7. Einführung des Plasmids pBINAR-ASAATP1 in das Genom von Kartoffelpflanzen

Die Übertragung des Plasmids erfolgt gemäß Punkt 5.
Als Ergebnis der Transformation zeigten transgene Kartoffelpflanzen eine Verringerung der mRNA eines plastidären ADP/ATP-Translokators. Dies wird mittels Northern Blot-Analyse nachgewiesen. Dazu wird RNA nach Standardprotokollen aus Blatt- und Knollengewebe von Kartoffelpflanzen isoliert. 50 µg RNA wurden auf einem Agarosegel aufgetrennt (1,5 % Agarose, 1 x MEN-Puffer, 16,6 % Formaldehyd). Nach der Elektrophorese wurde die RNA mit 20 x SSC mittels Kapillarblot auf eine Nylonmembran Hybond N (Amersham, UK) übertragen. Die RNA wird auf der Membran durch UV-Bestrahlung fixiert. Die Membran wird in Phosphathybridisierungspuffer (Sambrook et al., a.a.O) für 2 h prähybridisiert und anschließend durch Zugabe der radioaktiv markierten Sonde für 10 h hybridisiert.

### 8. Aminosäureanalytik

Die Aminosäuren (mit Ausnahme von Prolin) wurden durch HPLC-Auftrennung in ethanolischen Extrakten gemessen (nach Geigenberger et al., 1996, Plant Cell & Environ 19: 43-55).

### 8.1 Herstellung der ethanolischen Extrakte

Jeweils zwei Kartoffelscheiben (zusammen ca. 0.2 g Frischgewicht) werden in zwei aufeinanderfolgenden Schritten mit jeweils 7 ml 80 % (v/v) Ethanol und 7 ml 50 % Ethanol für 30 min. bei 80 °C extrahiert. Der Gesamtextrakt (Volumen ca. 14 ml) dient zur Bestimmung der Aminosäuren.

### 8.2 Bestimmung der Aminosäuregehalte durch HPLC

Der Nachweis der Aminosäuren erfolgt fluorometrisch nach Vorsäulenderivatisierung der primären Aminogruppe mit o-Phthalsäuredialdehyd (OPA). Hierzu injizierte ein Probengeber (Autosampler 465, Kontron, Eching) bei 4 °C in 35 µl vorgelegten Extrakt 35 µl OPA-Reagenz, bestehend aus einer Mischung von 5 % (g/v) OPA in Methanol, 0.8 M Boratpuffer (pH 10.4 mit KOH) und 3-Mercaptopropionsäure (10:90:1; v:v:v). Nach 108 Sekunden wurden 20 µl der derivatisierten Probe injiziert.
Laufmittel A ist eine Mischung von 1000 ml 12 mM Na-Phosphat (pH 6.8) und 1.6 ml Tetrahydrofuran. Laufmittel B besteht aus einer Mischung von 250 ml 12 mM Na-Phosphat (pH 6.8), 175 ml Methanol und 110 ml Acetonitril. Die Trennbedingungen sind wie folgt: 0-2 min, isokratische Phase mit 0 % B, 2-11 min., linearer Gradient von 0 auf 10 % B, 11-17 min., 10 % B, 17-27 min., linearer Gradient von 10 auf 50 % B, 27-38 min., linearer Gradient von 50 auf 60 % B, 38-44 min., linearer Gradient von 60 auf 100 % B, 44-46 min., 100 % B, 46-48 min., 100 auf 0 % B, 48-60 min., 0 % B. Zur Trennung wird ein Hypersil ODS-Säule (3 µm Partikelgröße, 150 mm Länge, 4.6 mm Durchmesser, Knauer GmbH, Berlin) verwendet. Die vom Fluorimeter (SFM25, Kontron, Eching) detektierten Signale (Excitationswellenlänge = 330 nm, Emissionswellenlänge = 450 nm) werden vom Datensystem 450-MT (Kontron, Eching) integriert und ausgewertet.

### 8.3 Bestimmung des Prolingehaltes

Die Bestimmung des Prolingehaltes erfolgt nach Bates et al., 1973, Plant Soil 39: 205-207.
Zu 200 µl Extrakt werden 500 µl einer Mischung von 2 Teilen 6 M H₃PO₄ und 3 Teilen 75 % Essigsäure, sowie 500 µl Ninhydrinlösung (600 mg pro 20 ml 75 % Essigsäure) zugegeben. Nach 45 min Inkubation bei 95-100 °C, wird der Testmix auf Eis gestellt und mit 300 µl Toluol vermischt. Nach erfolgter Phasentrennung wird die obere Phase in eine Microküvette transferiert und die OD bei 515 nm gemessen. Der Prolingehalt wird durch Vergleich mit einer Eichgeraden (1-50 µM Prolin) ermittelt.

## Patentansprüche

1. Verfahren zur Herstellung einer Pflanze mit erhöhtem Aminosäuregehalt, wobei ein Vektor enthaltend ein ATP/ADP-Translokator-Gen durch gentechnische Methoden in eine Pflanze oder Gewebe oder Zellen davon übertragen wird.

2. Verfahren nach Anspruch 1, **dadurch gekennzeichnet, dass** der ATP/ADP-Translokator eine für die in Fig. 1 angegebene Aminosäuresequenz kodierende Nukleotidsequenz aufweist.

## Claims

1. Process for the production of a plant having an increased content of amino acid, whereby a vector containing an ATP/ADP-translocator-gene is transferred into a plant or into tissue or into cells thereof by way of genetic methods.

2. Process according to Claim 1, **characterized in that** the ATP/ADP-translocator-gene has a nucleotide sequence encoding for the amino acid sequence as shown in figure 1.

## Revendications

1. Procédé pour la production d'une plante avec une teneur élévée d'aminoacide alors qu'un vecteur contenant un géne translocateur ATP/ADP est transféré dans une plante ou dans un tissu ou dans des cellules de cela par des methodes génétiques.

2. Procédé selon la revendication 1, **characterisé en ce que** le gène translocateur ATP/ADP comporte une séquence nucleotide codant pour la séquence aminoacide comme spécifiée dans la figure 1.
